# EUROPEAN PATENT APPLICATION

(11) **EP 2 206 482 A1**
(43) Date of publication of application: **14.07.2010**
(21) Application number: 10150641.8
(22) Date of filing: 13.01.2010
(51) Int. Cl.: A61F 2/08

(54) **Patch augmentation of achilles tendon repairs**

(30) Priority: 13.01.2009 US 144332 P
(71) Applicant: Tornier, Inc., Edina, MN 55435 (US)
(72) Inventor: Saltzman, Charles L., Salt Lake City, UT 84103 (US); Schon, Lew C., Baltimore, MD 21208 (US)
(74) Representative: Grand, Guillaume

(57) **Abstract**

A patch system for augmentation of an Achilles tendon repair, according to the present invention, comprises a patch comprising a proximal edge, a distal edge, a first side edge, and a second side edge. A first dimension of the patch between the proximal and distal edges is substantially the same as a distance between a calcaneus to which the Achilles tendon is attached and a location at which the Achilles tendon meets a soleus muscle, and a second dimension of the patch between the first and second side edges substantially corresponds to a medio-lateral width of the Achilles tendon. Besides, the patch is formed of a biocompatible material.

## Description

### TECHNICAL FIELD

Embodiments of the present invention relate generally to surgical repair of torn tendons and ligaments in an animal, and in particular to open and arthroscopic orthopedic surgical repair of torn tendons and ligaments in the body, such as repair of a torn or ruptured Achilles tendon.

### BACKGROUND

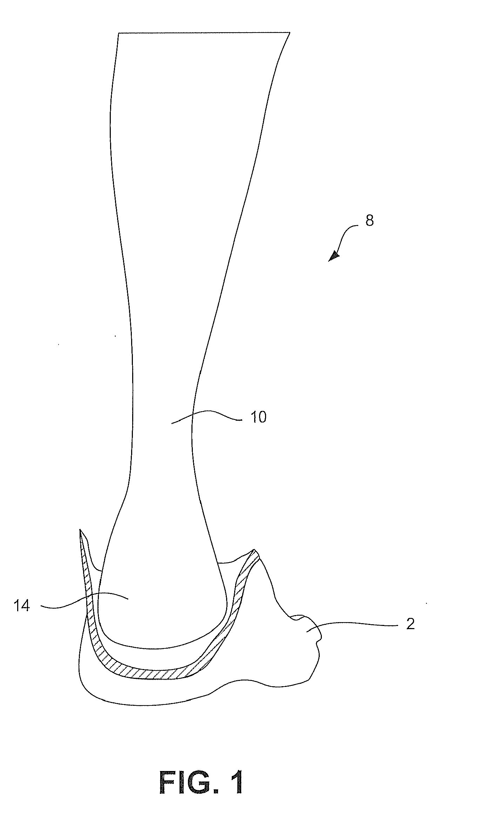

FIG. 1 is a posterior view of a patient's foot 2 and leg 8 including the Achilles tendon 10. The Achilles tendon 10 is the tendinous extension of three muscles in the lower leg: gastrocnemius, soleus, and plantaris. In humans, the tendon passes behind the ankle. It is the thickest and strongest tendon in the body. It is about fifteen centimeters long, and begins near the middle of the calf, but receives fleshy fibers on its anterior surface, almost to its lower end. Gradually becoming contracted below, it merges into the middle part of the posterior surface of the calcaneus 14.

Achilles tendon rupture is a partial or complete break in the tendon, and is the most common injury involving a break in a tendon. Rupture of the Achilles tendon 10 commonly occurs as a sports injury during explosive acceleration such as, for example, pushing off or jumping up.

Treatment for Achilles tendon ruptures can be non-surgical or surgical. The non-surgical approach to treatment typically involves the patient wearing a cast or walking boot, which allows the ends of the torn tendon to reattach themselves on their own. For some patients this method of treatment can be effective, and it avoids some of the risks associated with surgery such as, for example, infection. However, the likelihood of re-rupture is higher with a non-surgical approach, and recovery can take longer. If re-rupture occurs, surgical repair may be more difficult.

Surgery is a common treatment for a complete rupture of the Achilles tendon. One current surgical treatment approach involves wrapping a patch around the ruptured tendon after primary surgical repair of the tendon. The edges of the patch are sutured to form a tube and the tube is further sutured to the Achilles. Another surgical treatment approach involves suturing a patch along the posterior surface of the ruptured Achilles such that the patch spans the rupture to provide biological augmentation. In another treatment option, after primary repair of the Achilles, a tendon from the forefoot (FHL or FDL) is harvested, threaded through the proximal stump of the Achilles and then anchored to the calcaneus. In yet another surgical treatment approach, a bioimplant such as the OrthAdapt^{™} bioimplant is used instead of a tendon harvested from the FHL or FDL. In this approach, the implant is threaded through the Achilles much like the FHL would be and then subsequently anchored to the calcaneus.

### SUMMARY

The subject of the present invention is a patch system for an Achilles tendon repair, as defined in claim 1. Other advantageous features of this patch system are specified in dependent claims 2 to 9.

The present implant relieves at least part of the separation forces experienced by the repair during the recovery period, according to embodiments of the present invention. The implant may be absorbed by the body after healing. The implant may distribute the separation forces experienced in a ligament or tendon-to-bone surgical repair during the recovery period over a large area of the ligament or tendon. The implant may include reinforced regions in its construction to distribute attachment loads of sutures and to prevent sutures from tearing through the device, according to embodiments of the present invention.

In one embodiment, the implant mimics the elastic properties of natural tendon in order to allow a portion of anatomical loads to stress the tendon and prevent atrophy of the attached muscle. A suitable implant is described in U.S. Patent Application Serial No. 12/025,449, filed on February 4, 2008, entitled "System and Method for Repairing Tendons and Ligaments" and published on Aug. 7, 2008 as U.S. Patent Application Publication No. 2008/0188936.

In one embodiment, the implant conforms to the non-planar contours of an anatomical structure being repaired. The implant may be constructed in a shape that effectively applies reinforcement loads in the anatomically correct orientation for the body part being repaired.

In one embodiment, the implant includes an elongated body, and is preferably longer than conventional patches.

In one embodiment, the implant includes one or more anchor features to facilitate attachment to the tendon stump and/or calcaneus.

In one embodiment, the implant includes a flared distal and/or proximal end.

In one embodiment, the implant includes one or more anchors adapted to facilitate anchorage of the implant to a bone.

Implants according to embodiments of the present invention may be implanted using an open procedure or using minimally invasive arthroscopic surgical techniques.

In one embodiment, an implant method includes using locking sutures to anchor the patch in the proximal Achilles stump and anchoring the implant directly to the calcaneus. In one embodiment, an implant method includes extending the patch up to the muscle of the lower leg. In one embodiment, the method includes extending the patch down to the calcaneus.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 is a schematic, posterior view of a patient's foot and leg including the Achilles tendon.
FIG. 2 illustrates a front view of an augmentation patch, according to embodiments of the present invention.
FIG. 3 illustrates a front view of an augmentation patch that is flared at its distal and proximal ends, according to embodiments of the present invention.
FIG. 4 illustrates a front view of an augmentation patch that is flared at its distal and proximal ends, and whose distal end includes anchor features, according to embodiments of the present invention.
FIG. 5 illustrates a front view of an augmentation patch that is flared at its distal and proximal ends, and whose distal and proximal ends include anchor features, according to embodiments of the present invention.
FIG. 6 illustrates a posterior partial cross-sectional view of an augmentation patch attached to a ruptured Achilles tendon, according to embodiments of the present invention.
FIG. 7 illustrates a lateral view of an augmentation patch attached to a ruptured Achilles tendon, according to embodiments of the present invention.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

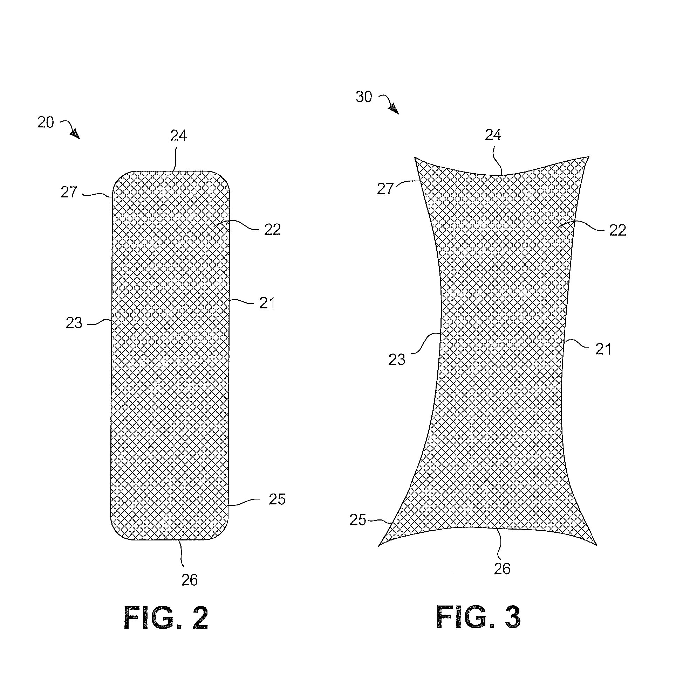

FIGS. 2-5 show augmentation patches 20, 30, 40, 50 used to repair a ruptured or partially ruptured Achilles tendon according to various embodiments of the present invention. The patch 20 provides a scaffold for biological ingrowth along the entire length of the tendon 10 without the need for tunneling through the tendon to thread or weave a tissue through the tendon 10. In use, the patch 20 mechanically augments the repaired Achilles tendon 10 by sharing the load placed on the Achilles. Additionally, the patch 20 biologically augments the repaired Achilles without the need to harvest the flexor hallucis longus tendon (FHL) or the flexor digitorum longus tendon (FDL) or other autologous tissues. Less peritenon is stripped such as in, for example, a wrap procedure, according to embodiments of the present invention. This results in less disruption of the blood supply, which may lead to better healing of the repaired Achilles tendon, according to embodiments of the present invention. Finally, the patch 20 provides no additional bulk between the Achilles and the overlying skin, thereby avoiding potential skin healing failures, according to embodiments of the present invention.

In one embodiment, the patch 20 is configured to conform to the non-planar contours of an anatomical structure being repaired. The implant may be constructed in a shape that effectively applies reinforcement loads in the anatomically correct orientation for the body part being repaired. The augmentation patch 20 is made from a patch material 22 and includes a proximal edge 24, a distal edge 26, a first side edge 21, and a second side edge 23. According to some embodiments of the present invention, the proximal and distal edges 24, 26 and the first and second side edges 21, 23 are reinforced. According to some embodiments, the proximal and distal edges 24, 26 and the first and second side edges 21,23 are reinforced to resist suture tear-out and to increase strength.

In one embodiment, reinforcing the patch 20 includes welding of the patch material 22 along one or more of the edges. The welding can be performed with various types of energy, such as, for example, ultrasonic, laser, electrical arc discharge, or thermal energy. In another embodiment, multiple layers of patch material 22 are attached to one or more of the edges, such as by adhesive, welding, or mechanical fasteners. In another embodiment, additional layers of patch material 22 and/or tension members are woven or knitted along the edges. In some embodiments, sutures can be used to distribute tendon loads to the bone, through the side edges 21, 23 by use of high strength tension members arranged along the preferred load direction.

The patch material 22 and tension members may be the same or different material/structure. For example, the patch material may be biocompatible and/or bioabsorbable, while the tension members may include a non-absorbable component. In another embodiment, the patch material and/or the tension members are a composite of synthetic material and natural material, such as for example an allograft or xenograft material.

According to embodiments of the present invention, the patch material 22 and/or tension members are multiple component materials. The different materials may each have a different melting point. The patch material and/or tension members can be composed of a single filament or multiple filaments. The filaments can be homogenous or heterogeneous. When multiple filaments are present, the material composition of the filaments can vary from filament to filament. Multiple filaments can include a mixture of both single-material filaments and multi-material filaments. The patch material and/or tension members may be a single strand of multiple fibers or it can include multiple strands. When multiple strands are included, these may be twisted together, braided, and/or otherwise interlinked, such as in a sheath-and-core configuration. A composite material that may be used according to embodiments of the present invention is described in U.S. Patent Application Serial No. 11/349,851, filed on June 5, 2006, and published on January 25, 2007 as U.S. Patent Application Publication No. 2007/0021780.

The structure of the patch material 22 and/or tension members can be one or more layers of the same or different materials, such as, for example, woven mesh, non-woven mesh (such as, for example, non-woven mesh that is melt-blown, hydro-entangled, and the like), multifilament mesh, monofilament mesh, terrycloth, fabric made by weaving, knitting, braiding and/or felting fibers, film, or any combination or composites thereof. Patch material 22 and tension members may also be autologous, allogeneic, or xenogeneic tissues, according to embodiments of the present invention.

According to some embodiments of the present invention, the tension members are a single filament such as a monofilament, or a grouping of a plurality of pliable, cohesive threadlike filaments (e.g. a braided suture), or an elongated section of woven or non-woven fabric or mesh.

The patch material 22 and the tension members may be made of an absorbable or bioabsorbable material. As used herein, the terms "absorbable" or "bioabsorbable" are used in their broadest sense to mean the complete degradation of a material in vivo, and elimination of its metabolites from an animal or human. According to some embodiments of the present invention, the patch material 22 may be made with a polyglycolic acid, a polydioxanon material, and/or poly-L-lactic acid.

While the patch material 22 and tension members may be constructed from an absorbable material, one or both may be reinforced by non-absorbable materials, including without limitation glass fibers, natural fibers, carbon fibers, metal fibers, ceramic fibers, synthetic or polymeric fibers, composite fibers (such as a polymeric matrix with a reinforcement of glass, natural materials, metal, ceramic, carbon, and/or synthetics components), or a combination thereof. In one embodiment, the tension members are relatively stiff and the implant does not distort in response to oblique loads. In other embodiments, the tension members are somewhat elastic and the implant distorts under oblique loads.

In one embodiment, the tension members and patch material 22 comprise a woven structure that can be trimmed to shape with minimal or no unraveling or fraying along the cut edge. Fraying reduces mechanical strength and suture retention ability. Loose fibers can migrate and provoke an inflammatory reaction. Consequently, special textile manufacturing techniques may be used to prevent these problems.

In one embodiment, the patch material and/or tension members are constructed using a "leno" weave. A standard weave has an array of warp fibers in one direction that run alternately over and under weft fibers in the perpendicular direction. In a leno weave, pairs of warp fibers wrap over and under each weft fiber and then across each other to lock each fiber in place. Leno weaves are much more resistant to unraveling when cut. Leno weaves are also more porous and allow tissue in-growth better than plain weaves, according to embodiments of the present invention.

In another embodiment, the tension members and/or patch material 22 comprise a weave constructed on a shuttle loom. In modern, conventional weaves the weft fibers run across the fabric and end at the edges. With a shuttle loom the weft fiber is woven across the fabric and then it turns around and weaves back across the fabric as a continuous fiber. The edges are thus much more stable. As used herein, "scalable weave" refers to a textile structure that can be trimmed with minimal or no unraveling along cut edges, such as for example by a leno weave or textiles made using a shuttle loom.

While the scalable weaves discussed above may be infinitely scalable (e.g, the surgeon can cut anywhere with minimal risk of fraying or unraveling), in some embodiments the patch material 22 and/or tension members are reinforced to be incrementally scalable (e.g., the surgeon can cut along pre-determined cut lines). Pre-determined cut lines can be formed by welding, adding a resin to the fabric, attaching one or more reinforcing layers, or combinations thereof. The surgeon can cut the patch material and/or tension members along the pre-determined cut lines to fit a particular patient with minimal risk of fraying or unraveling.

According to some embodiments of the present invention, the patch material 22 and the tension members are made of a slow-absorbing, biologically benign material, such as Poly-4-hydroxybutyrate (also known as Tephaflex^{™}), poly(urethane urea) (Artelon^{™}), silk, polymers containing lactide, glycolide, caprolactone, trimethylene carbonate, dioxanone, or other materials known to one of ordinary skill in the art as having similar characteristics, such as those described in U.S. Patent Application Publication No. 2007/0198087, entitled Method and Device for Rotator Cuff Repair, published on August 23, 2007, and U.S. Patent Application Publication No. 2007/0276509, entitled Tissue Scaffold, published on November 29, 2007. A patch material 22 according to other embodiments employs non-absorbable materials such as PTFE, Polyester, Polypropylene, Nylon, PEEK, or other biocompatible, inert materials known to one of ordinary skill in the art. In some embodiments, monofilaments may be used in combination with weaves, knits, braids, and the like, to increase porosity for tissue in-growth, increase abrasion resistance, and give selective tensile strength along a load direction. In another embodiment, the patch material 22 is a Conexa^{™} strip or reconstructive tissue matrix. Alternatively, the implant 20 may be constructed from xenograft and/or allograft materials, according to embodiments of the present invention.

Immediately after surgery, the patch 20 carries the majority of the anatomical load, according to embodiments of the present invention. During the course of healing, which is usually twelve to twenty-six weeks, the tendon-to-bone repair gains strength while the augmentation patch 20 loses strength and is absorbed by the body.

Rotator cuff tendons are also commonly repaired. While the surgical repair has historically been performed as an open procedure (and more recently as a "mini-open" repair), the majority of rotator cuff repairs are now done fully arthroscopically, with the tendon being reattached directly to the bony insertion on the lateral border of the humerus. However, when direct reattachment is not possible, for example, because retraction of the muscle has created a large defect, interposition implants or grafts (including synthetic cuff prostheses) are used to fill the gap formed by the defect. Implants (or grafts) are also used as augmentation implants to strengthen a repair to prevent recurrent tears and allow for a more aggressive rehabilitation particularly in younger patients. Augmentation implant (or graft) refers to a material that can be used to strengthen a tendon or ligament. For example, a surgeon may enhance the strength of a rotator cuff repair made with sutures by incorporating a reinforcing material into the repair. Interposition implant (or graft) refers to a material that is used to bridge a gap (or defect) between the end of a tendon and its bony insertion site. An implant may be an interposition implant and/or an augmentation implant, according to embodiments of the present invention. The patch 20 can be attached firmly to the rotator cuff tendons proximally to the repair site and then anchored directly to the humeral head so that the patch is in intimate contact with the distal tendon to encourage tissue ingrowth into the patch.

In some embodiments, as shown in FIG. 2, the patch 20 is an elongated strip of the patch material 22. In other embodiments, as illustrated in FIG. 3, the patch 30 is an elongated strip of patch material 22 including at least one flared end 25, 27. Patch 30 may include any of the characteristics of patch 20 described herein, according to embodiments of the present invention. According to embodiments of the present invention, distal end 35 is flared; according to other embodiments of the present invention, proximal end 37 is flared; according to yet other embodiments of the present invention, both distal end 35 and proximal end 37 are flared, as shown in FIG. 3.

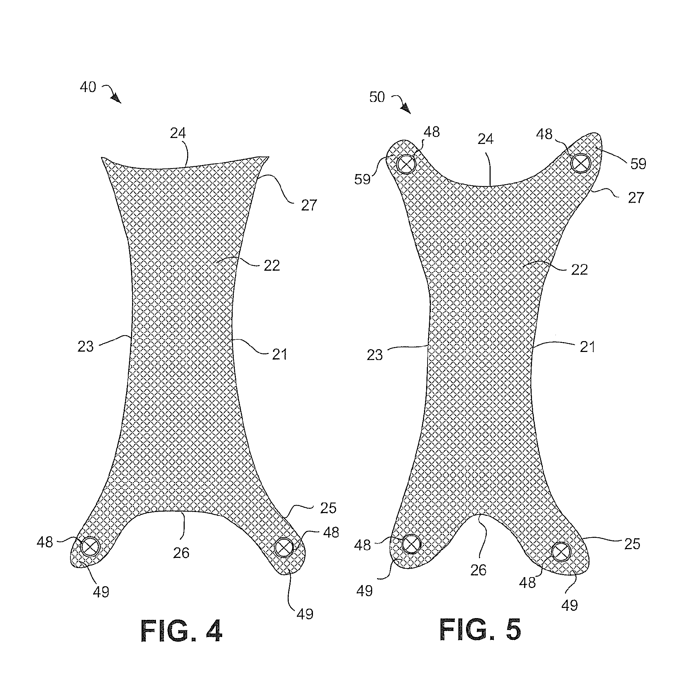

As illustrated in FIG. 4, a patch 40 may include a proximal end 27 that is flared, as well as a distal end 25 that is flared and includes one or more anchor features 49, according to embodiments of the present invention. An anchor feature 49 may be a protrusion, bulge, and/or stretchable corner of the patch 40, according to embodiments of the present invention. Patch 40 may include any of the characteristics of patch 20 described herein, according to embodiments of the present invention. Anchor features 49 may facilitate anchoring of the patch 40 to the calcaneus 14. In one embodiment, the distal end 25 of the patch 40 includes a pair of anchors features 49. The anchors features 49 may extend along the sides of the calcaneus 14 to anchor the patch 40 the bone, according to embodiments of the present invention.

In another embodiment, as shown in FIG. 5, a patch 50 includes one or more distal anchor features 49 and one or more proximal anchor features 59, according to embodiments of the present invention. Patch 50 may include any of the characteristics of patch 20 described herein, according to embodiments of the present invention. In addition, one or more of the proximal end 27 and distal end 25 of patch 50 may be flared, according to embodiments of the present invention. Proximal anchor features 59 may be configured to facilitate attachment of the patch 50 to the soleus muscle 72, according to embodiments of the present invention.

According to embodiments of the present invention, the distal end 25 and distal edge 26 include anchors 49 that resist tear-out of any sutures attached to the bone through the patch 40, 50. In addition to sutures, other attachment mechanisms can be used to secure the distal edge 26 and anchors 49 to the calcaneus 14. Exemplary attachment means include attachment mechanisms such as trans-tendon anchors, bone anchors, darts, screws, glue, tacks, staples, or any combination thereof. Various attachment mechanisms suitable for attaching the patch 20, 30, 40, 50 to the calcaneus 14 are described in U.S. Pat. Nos. 6,923,824, issued August 2, 2005; 6,666,877, issued December 23, 2003; 6,610,080, issued August 26, 2003; 6,056,751, issued May 2, 2000; and 5,941,901, issued on Aug. 24, 1999. As illustrated in FIGS. 4 and 5, the anchor features 49, 59 optionally include a pre-formed opening or eyelet 48 adapted to receive sutures or to engage with another anchoring means, such as, for example, a bone anchor. The term eyelet refers to a preformed opening, preferably round and finished along the edge.

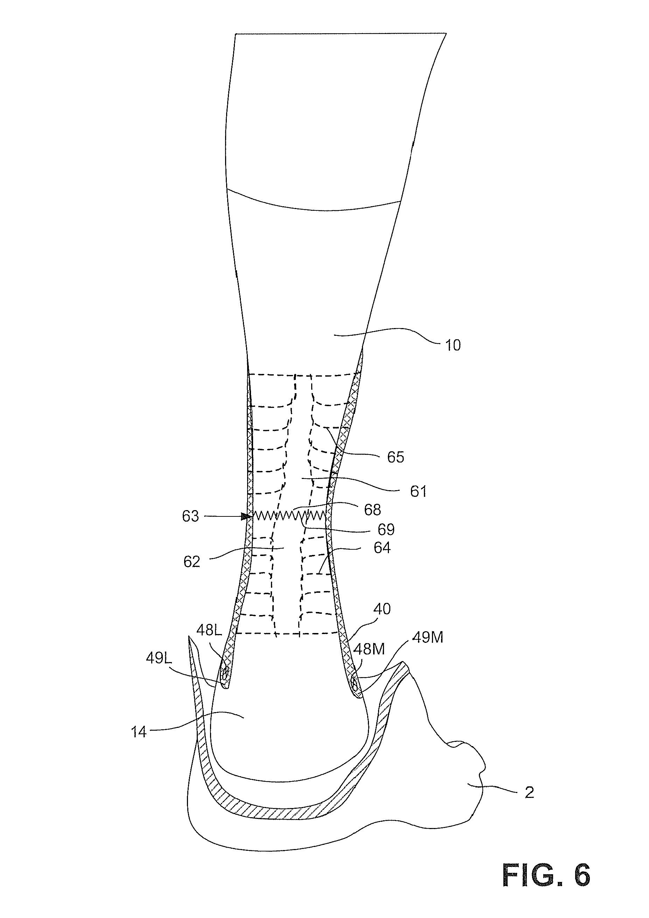

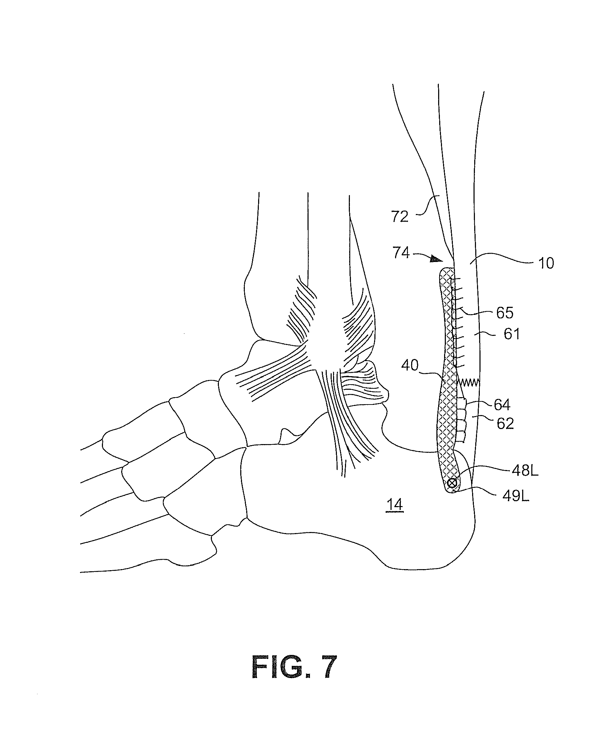

FIG. 6 is a posterior view of a patch 40 used to repair a ruptured Achilles tendon 10, according to embodiments of the present invention. FIG. 7 is a side view of the ruptured Achilles tendon 10 including the patch 40 shown in FIG. 6, according to embodiments of the present invention. The Achilles 10 includes a rupture at location 63, including a proximal stump 61 and a distal stump 62, according to embodiments of the present invention. According to various embodiments of the present invention, the patch 40 is secured to the ruptured Achilles tendon 10, such that the ends 68, 69 of the proximal and distal stumps 61, 62 are brought together such that substantially no gap exists between each of their ends 68, 69. Although patch 40 is illustrated, various other patches 20, 30, 50 shown and described may be used to augment the Achilles tendon repair, according to embodiments of the present invention.

First, according to one method, a plurality of suture stitches 64 such as, for example, Krackow stitches are placed in the distal stump 62 of the Achilles tendon 10. Other stitches or stitch patterns may be used, for example, lateral trap and/or Kessler stitches. Next, the patch 40 is positioned under the ruptured tendon 10 such that it extends from about the soleus muscle 72 to the calcaneus 14, according to embodiments of the present invention. Additional suture stitches 65 such as, for example Krackow stitches, are secured in the proximal stump 61 of the Achilles tendon 10 such that each suture stitch 65 captures the patch 40, according to embodiments of the present invention. According to other embodiments of the present invention, only some of the suture stitches 65 capture the patch 40. The suture ends are then used to tie both set of sutures 64, 65 such that the ends 68, 69 of the proximal stump 61 and the distal stump 62 are apposed without a gap between them, according to embodiments of the present invention. The distal end 26 of the patch 40 is then anchored to the calcaneus 14 such that it shares a large portion of the load placed on the Achilles tendon 10. In one embodiment, the central region of the distal end 26 of the patch 40 is trimmed away such that it does not impinge on and/or interfere with the Achilles insertion region. Additional sutures, such as for example, stay sutures (not shown), can be used as necessary to promote intimate contact between the distal stump 62 of the Achilles tendon 10 and the patch 40, such that biological augmentation of the distal stump 62 is encouraged.

Embodiments of the present invention include methods for repairing an Achilles tendon 10 that has completely or partially ruptured leaving a distal stump 62 extending from a calcaneus 14 and a proximal stump 61, the proximal stump 61 having a proximal stump end 68, and the distal stump 62 having a distal stump end 69. Methods according to such embodiments include attaching a first suture 64 to the distal stump 62, positioning an augmentation patch 40 against an anterior portion of the Achilles tendon 10, attaching a second suture 65 to the proximal stump 61 and to the augmentation patch 40, apposing the proximal stump end 68 and the distal stump end 69 such that a gap between the proximal stump end 68 and distal stump end 69 is minimized or eliminated, joining the first suture 64 with the second suture 65 to secure the proximal stump 61 to the distal stump 62, and attaching the augmentation patch 40 to the calcaneus 14. According to embodiments of the present invention, the first suture 64 is not attached to the augmentation patch 40.

According to embodiments of the present invention, the first suture 64 has at least one first free end after attachment to the distal stump 62 and the second suture 65 has at least one second free end after attachment to the proximal stump 61 and the augmentation patch 40. In such cases, joining the first suture 64 with the second suture 65 can include tying together the first free end and the second free end.

The augmentation patch 40 may include a proximal end 27 and a distal end 25, and positioning the augmentation patch 40 includes positioning the distal end 25 at the calcaneus 14 and positioning the proximal end 27 at a location 74 where the Achilles tendon 10 meets a soleus muscle 72, according to embodiments of the present invention. Attaching the first suture 64 to the distal stump 62 may include passing the first suture 64 through the distal stump 62 such as, for example, with a Krackow stitch pattern. Attaching the second suture 65 to the proximal stump 61 and the augmentation patch 40 may include passing the second suture 65 through the proximal stump 61 and the augmentation patch 40 such as, for example, with a Krackow stitch pattern. According to embodiments of the present invention, each stitch of the Krackow stitch pattern passed through the proximal stump 61 captures the augmentation patch 40.

The augmentation patch 40 may be flared at the distal end for attachment to the calcaneus 14. According to embodiments of the present invention, the distal end 25 includes a medial anchoring feature 49M and a lateral anchoring feature 49L, and attaching the augmentation patch 40 to the calcaneus 14 includes attaching the augmentation patch 40 to the calcaneus 14 at the medial and lateral anchoring features 49M, 49L. According to embodiments of the present invention, the medial anchoring feature 49M includes a medial eyelet 48M and/or the lateral anchoring feature 49L includes a lateral eyelet 48L.

A patch system according to embodiments of the present invention includes a patch, one or more sutures, and/or one or more bone anchors. The patch 40 may include a proximal edge 24, distal edge 26, first side edge 21, and second side edge 23. A first dimension of the patch 40 between the proximal and distal side edges 21, 23 may be substantially the same as a distance between a calcaneus 14 to which the Achilles tendon 10 is attached and a location 74 at which the Achilles tendon 10 meets a soleus muscle 72, according to embodiments of the present invention. A second dimension of the patch between the first and second side edges 21, 23 substantially corresponds to a medio-lateral width of the Achilles tendon 10, according to embodiments of the present invention.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. A patch system for augmentation of an Achilles tendon repair, the patch system comprising:
a patch comprising:
a proximal edge;
a distal edge;
a first side edge;
a second side edge;
wherein a first dimension of the patch between the proximal and distal edges is substantially the same as a distance between a calcaneus to which the Achilles tendon is attached and a location at which the Achilles tendon meets a soleus muscle, and wherein a second dimension of the patch between the first and second side edges substantially corresponds to a medio-lateral width of the Achilles tendon; and
wherein the patch is formed of a biocompatible material.

2. The patch system of claim1, further comprising:
a first suture configured for placement through a distal stump of the Achilles tendon; and
a second suture configured for placement through both the patch and a proximal stump of the Achilles tendon.

3. The patch system of claim 2, further comprising at least one bone anchor configured to attach the patch to the calcaneus near the distal edge.

4. The patch system of any one of claims 1 to 3, wherein at least one of the proximal edge and the distal edge is flared.

5. The patch system of claim 4, wherein both the proximal edge and the distal edge are flared.

6. The patch system of any one of claims 1 to 5, wherein the patch includes an anchor feature.

7. The patch system of any one of claims 1 to 6, wherein the patch includes a first anchor feature proximate to a medial corner of the distal edge and a second anchor feature proximate to a lateral corner of the distal edge, and wherein the first and second anchor features are configured to facilitate attachment of the patch to the calcaneus.

8. The patch system of claim 7, further comprising:
a medial bone anchor configured for insertion into the calcaneus through the first anchor feature; and
a lateral bone anchor configured for insertion into the calcaneus through the second anchor feature.

9. The patch system of any one of claims 1 to 8, wherein the patch is formed of a scalable weave material.
